(19) Europäisches Patentamt
European Patent Office
Office européen des brevets

(11) **EP 4 728 979 A1**

(12) **EUROPEAN PATENT APPLICATION**

(43) Date of publication:
**22.04.2026 Bulletin 2026/17**

(21) Application number: **25208548.5**

(22) Date of filing: **14.10.2025**

(51) International Patent Classification (IPC):
**A61B 5/024** (2006.01)  **A61B 5/11** (2006.01)
**A61B 5/1455** (2006.01)  **A61B 5/00** (2006.01)
**G16H 40/63** (2018.01)  **G16H 50/20** (2018.01)

(52) Cooperative Patent Classification (CPC):
**A61B 5/02416; A61B 5/14551; A61B 5/681;**
**A61B 5/7267; A61B 5/746; G16H 50/20;**
A61B 5/1118; A61B 2562/0219; G16H 40/63

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **15.10.2024 US 202463707584 P**

(71) Applicant: **GOOGLE LLC**
**Mountain View CA 94043 (US)**

(72) Inventors:
• **SUNSHINE, Jacob Eugene**
**Mountain View, 94043 (US)**

• **GADH, Tajinder Singh**
**Mountain View, 94043 (US)**
• **STANGE, Anthony Michael**
**Mountain View, 94043 (US)**
• **SHAH, Kamal Girish**
**Mountain View, 94043 (US)**
• **WANG, Anran**
**Mountain View, 94043 (US)**
• **PATHAK, Anupam J.**
**Mountain View, 94043 (US)**

(74) Representative: **Maikowski & Ninnemann**
**Patentanwälte Partnerschaft mbB**
**Postfach 15 09 20**
**10671 Berlin (DE)**

(54) **OXYGEN EMERGENCY DETECTION FOR A WEARABLE COMPUTING DEVICE**

(57) A wearable computing device includes one or more sensors for detecting low motion of a user of the wearable computing device and a processor communicatively coupled with the sensor(s). The processor is configured to perform a plurality of operations, including, in response to detecting the low motion of the user, monitoring an oxygen saturation level of the user for a drop from above an upper threshold to consistently below a lower threshold, upon detecting the oxygen saturation level dropping from above the upper threshold to consistently below the lower threshold, determining an oxygen saturation drop above a certain magnitude, in response to the oxygen saturation drop being above the certain magnitude, requesting an input from the user via the wearable computing device, and implementing, via the wearable computing device, an emergency response if the input is not received.

*FIG. 1A*

**Description**

FIELD OF THE INVENTION

The present disclosure relates generally to wearable computing devices, and more particularly to oxygen emergency detection for a wearable computing device.

BACKGROUND

Unanticipated drops in oxygenation that do not recover or self-correct represent time sensitive emergencies and can arise from a number of causes - most commonly opioid toxicity accidents. Early detection and rapid intervention can prevent death from these accidents. At high doses, opioids (particularly fentanyl) can cause rapidly diminished or complete cessation of breathing, hypoxemic/hypercarbic respiratory failure, and death, the physiologic sequence by which people commonly succumb to unintentional opioid toxicity accidents. Unlike many life-threatening medical emergencies, opioid toxicity is readily reversed with rapid identification and administration of the overdose antidote naloxone or supportive respiratory care. A fundamental challenge of fatal toxicity accidents is that victims die alone or among untrained or impaired bystanders, with insufficient timely identification and intervention. Moreover, existing over-the-counter vital sign monitors are not effective in detecting such events because their design discourages continuous use, thereby potentially leading to missed events. In addition, some individuals may be deterred from using these monitors due to their association with opioid use and perceived stigmatization. Furthermore, existing monitors cannot differentiate between less urgent critical oxygen drops that recover and lack the ability to contact emergency services.

Impaired oxygenation events that are emergent typically start in a low motion state with a decrease in the blood O2 saturation level. The continuous lack of oxygenation intake further reduces the O2 saturation level fairly rapidly (e.g., over a duration of minutes). If the O2 saturation drops to a dangerous level (in this example below 83%) and persists without recovery, the persistently low O2 state can lead to brain injury, cardiac arrest, and death.

Thus, there is an unmet need for identifying people experiencing oxygen emergency events, particularly in unwitnessed settings, and connecting them to help sooner. Accordingly, to help address this unmet need, and to help connect potential victims with widely available life-saving interventions, a wearable computing device equipped with oxygen emergency detection would be welcomed in the art.

SUMMARY OF THE INVENTION

Aspects and advantages of embodiments of the present disclosure will be set forth in part in the following description, or can be learned from the description, or can be learned through practice of the embodiments.

In an aspect, the present disclosure is directed to a wearable computing device. The wearable computing device includes one or more sensors for detecting low motion of a user of the wearable computing device and a processor communicatively coupled with the one or more sensors. The processor is configured to perform a plurality of operations including in response to detecting the low motion of the user, monitoring an oxygen saturation level of the user for a drop from above an upper threshold to consistently below a lower threshold, upon detecting the oxygen saturation level dropping from above the upper threshold to consistently below the lower threshold, determining an oxygen saturation drop above a certain magnitude, in response to the oxygen saturation drop being above the certain magnitude, requesting an input from the user via the wearable computing device; and implementing, via the wearable computing device, an emergency response if the input is not received.

In another aspect, the present disclosure is directed to a method of detecting and responding to an oxygen emergency of a user of a wearable computing device. The method includes detecting low motion of the user via the wearable computing device. The method also includes, in response to detecting the low motion of the user, monitoring, via the wearable computing device, an oxygen saturation level of the user for a drop from above an upper threshold to consistently below a lower threshold. Further, the method includes, upon detecting the oxygen saturation level dropping from above the upper threshold to consistently below the lower threshold, determining, via the wearable computing device, an oxygen saturation drop above a certain magnitude. In response to the oxygen saturation drop being above the certain magnitude, the method includes requesting an input from the user via the wearable computing device. Moreover, the method includes implementing, via the wearable computing device, an emergency response if the input is not received.

In yet another aspect, the present disclosure is directed to a method of detecting and responding to an oxygen emergency of a user of a wearable computing device. The method includes receiving, via one or more sensors of the wearable computing device being worn by the user, sensor data relating to an oxygen saturation level of the user and determining, via a processor of the wearable computing device, an occurrence of the oxygen emergency based on the sensor data.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

BRIEF DESCRIPTION OF THE DRAWINGS

Detailed discussion of embodiments directed to one of ordinary skill in the art is set forth in the specification, which makes reference to the appended figures, in which:

FIG. 1A illustrates a front perspective view of a wearable computing device on a wrist of a user according to the present disclosure;
FIG. 1B illustrates a side view of the wearable computing device of FIG. 1A according to the present disclosure;
FIG. 2 illustrates a schematic diagram of an example system that can be utilized with the wearable computing device of FIGS. 1A-1B according to the present disclosure;
FIG. 3 illustrates a schematic diagram of an embodiment of phase progression of oxygen emergency detection for a wearable computing device according to the present disclosure;
FIG. 4 illustrates an example graph of a tri-class machine-learned (ML) model with tri-class probabilities (i.e., low, medium, and high O2Sat values) along time for an O2Sat drop session according to the present disclosure;
FIG. 5 illustrates an example graph of reflected light for green, red, and infrared photoplethysmography (PPG) signals, and how such signals change with every heartbeat according to the present disclosure;
FIG. 6 illustrates an embodiment of a graph of a measured PPG signal versus time according to the present disclosure, particularly illustrating different extracted components of the measured PPG signal;
FIG. 7 illustrates an example graph of O2Sat as a percentage (%) versus time according to the present disclosure;
FIG. 8 illustrates a flow diagram of an embodiment of various, detailed algorithmic stages of an oxygen emergency detection system according to the present disclosure;
FIG. 9 illustrates a flow diagram of an embodiment of a sleep gating algorithm of an oxygen emergency detection system according to the present disclosure;
FIG. 10 illustrates a schematic diagram of an embodiment of a system of detecting and responding to an oxygen emergency of a user of a wearable computing device according to the present disclosure; and
FIG. 11 illustrates a flow diagram of an embodiment of a method of detecting and responding to an oxygen emergency of a user of a wearable computing device according to the present disclosure.

DETAILED DESCRIPTION

Reference now will be made in detail to embodiments of the present disclosure, one or more examples of which are illustrated in the drawings. Each example is provided by way of explanation of the present disclosure, not limitation of the present disclosure. In fact, it will be apparent to those skilled in the art that various modifications and variations can be made in the present disclosure without departing from the scope of the independent claims. For instance, features illustrated or described as part of one embodiment can be used with another embodiment to yield a still further embodiment. Thus, it is intended that the present disclosure covers such modifications and variations as come within the scope of the appended claims and their equivalents.
Impaired oxygenation events that are emergent typically start in a low motion state with a decrease in the blood O2 saturation level. The continuous lack of oxygenation intake further reduces the O2 saturation level fairly rapidly (e.g., over a duration of dozens of seconds to minutes). If the O2 saturation drops to a dangerous level (as an example, below 83%) and persists without recovery, the persistently low O2 state can lead to brain injury, cardiac arrest, and death.
Thus, the present disclosure is directed to an oxygen emergency detection system implemented by a wearable computing device having PPG sensors and accelerometers such that the system can detect life-threatening O2 saturation drops within this latency, with a reasonably high sensitivity and sufficiently low specificity that would not affect daily usage for healthy users. In addition, the oxygen emergency detection system can distinguish between chronic hypoxia (such as COPD and altitude hypoxia) and recoverable short-term hypoxia (such as sleep apnea).
Accordingly, to help address this unmet need, and to help connect potential victims with widely available life-saving interventions, the oxygen emergency detection system can passively (i.e., without user input) identify physiological signs of oxygen emergencies and place a communication to close contacts and/or to a (statutory or otherwise predefined) emergency telephone number, such as 911 or 112, and/or one or more persons in a close proximity to the user. In particular embodiments, for example, the oxygen emergency detection system of the present disclosure includes several stages. In an embodiment, the system first identifies whether the input sensor data satisfies signal quality requirements, including a check for low motion via one or more accelerometer sensors. The system can then opportunistically activate additional red, infrared, and/or any other PPG channels to identify periods of persistently low oxygen saturation that are suggestive of impaired oxygenation levels. Then, a subsequent stage can confirm that the persistently low oxygen saturation levels are accompanied by an acute drop in oxygen saturation, and that the oxygenation state is not trending upward/recovering.

Accordingly, the combination of both persistently low absolute oxygen saturation levels, a large relative change (e.g., a large drop) in oxygen saturation levels, and a low motion state ensures that the system is performant in individuals of diverse skin pigmentation and minimizes false positives. In addition, in an embodiment, the system may include one or more signal quality and/or final check algorithms (such as a breathing interference check, a hypoxia probability check, and/or a test-time signal reversal check) to reduce false positives. Moreover, in an embodiment, the present disclosure uses a combination of digital signal processing (DSP) and machine-learning to identify impaired oxygenation levels. Furthermore, in an embodiment, the oxygen emergency detection system may be an opt-in feature and can be off by default. Thus, in an embodiment, the oxygen emergency detection system can first ensure that one or more pre-conditions are met (such as sensor data being available, the wearable computing device being on-wrist, and the user opting to use the oxygen emergency detection feature, etc.), after which the system can be run.

Moreover, in an embodiment, the system can include sleep gating in which the system can pause when the user has been persistently asleep in the recent past. Furthermore, in an embodiment, if an oxygen emergency is not occurring, the user can engage the wearable computing device, e.g., via the screen, a button, or a tap, to cancel further action. In addition, the system can include a motion cancellation feature (e.g., the user can move his arm to cancel). In such embodiments, further action is cancelled simply by user movement, rather than requiring the user to engage a screen, button, etc. of the wearable computing device.

With reference now to the Figures, example embodiments of the present disclosure will be discussed in further detail.

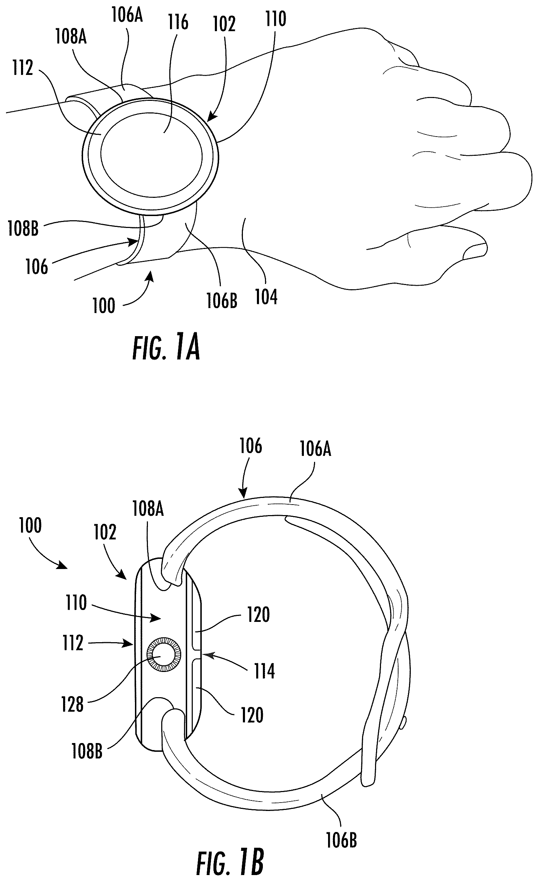

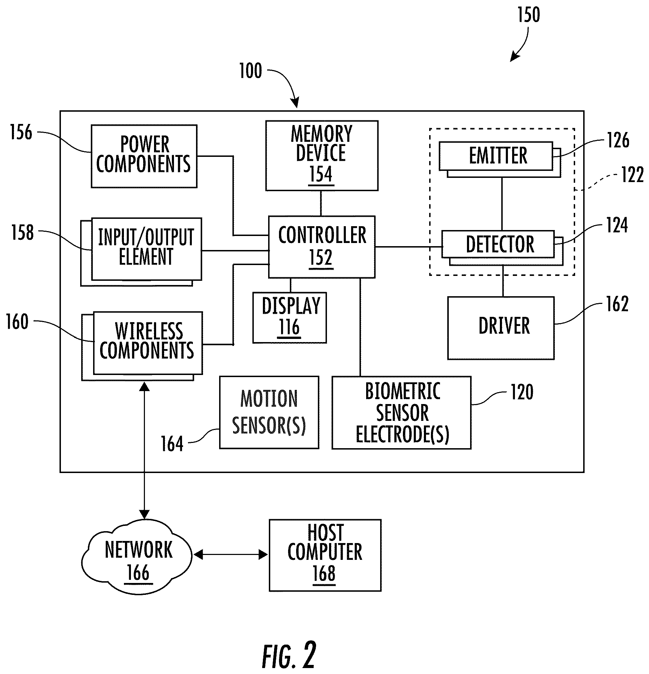

Referring now to the drawings, FIGS. 1A and 1B illustrate perspective views of a wearable computing device 100 according to the present disclosure and FIG. 2 illustrates an example system 150 that can be utilized with such wearable computing device 100. In general, the wearable computing device 100 includes a housing 102 that contains the electronics associated with the wearable computing device 100. The housing 102 is configured to rest against a user. In some instances, as shown in FIG. 1A, the wearable computing device 100 may be worn on a user's forearm 104 like a wristwatch. Thus, as shown, the wearable computing device 100 may include a wearable band, such as a wristband 106 having one or more parts, such as the two wristband straps 106A, 106B, for securing the wearable computing device 100 to the user's forearm 104. In such an instance, the housing 102 may include one or more connection points (e.g., connection points 108A, 108B) for such wristband 106. However, it should be appreciated that the wearable computing device 100 may be worn at any other suitable location by a user, such as, for example, on an ankle.

In addition, as shown in FIGS. 1A-1B, the housing 102 has a main housing 110, an upper housing cover 112, and a lower housing cover 114 (FIG. 1B). As particularly shown in FIG. 1B, the main housing 110 of the housing 102 of the wearable computing device 100 generally extends between the upper housing cover 112 and the lower housing cover 114 of the housing 102. As will be described in greater detail below, the upper and lower housing covers 112, 114 are connectable to the main housing 110 to seal an interior volume of the wearable computing device 100. In some instances, the connection points 108A, 108B may be provided on the main housing 110.

The upper housing cover 112 is configured to receive an electronic display screen 116. For example, in an embodiment, the upper housing cover 112 may be constructed of glass, polycarbonate, acrylic, or similar. The electronic display screen 116 may be arranged within the housing 102 and viewable through the upper housing cover 112. Moreover, in an embodiment, the electronic display screen 116 may cover an electronics package (not shown), which may also be housed within the housing 102.

The lower housing cover 114 of the housing 102 may be configured to be closest to a user when worn. For instance, the lower housing cover 114 may contact a dorsal wrist of a user when being worn by the user. As such, the plurality of sensor electrodes 120 may be positioned on the lower housing cover 114 so as to maintain skin contact with the user when being worn by the user. Thus, in such embodiments, each of the sensor electrodes 120 may be configurable to measure, at least, electrical impedance of the user at a location of the skin contact (e.g., at the dorsal wrist). Accordingly, in one or more embodiments, one or more (or all) of the plurality of sensor electrodes 120 may be impedance sensor electrodes.

Further, the sensor electrodes 120 described herein may be constructed of any suitable material. For example, in an embodiment, the sensor electrodes 120 described herein may be constructed of stainless steel, graphene, or any other material having a suitable conductivity and/or corrosion resistance and may have an optional PVD coating, that may be 1-micrometer thick titanium nitride. In such embodiments, the PVD coating may provide a desired color to the sensor electrodes 120, thereby preventing oxidation beyond what the stainless steel already provides, and also increases durability. In additional embodiments, PVD and surface finish can be used to increase/decrease moisture retention, which affects the impedance signal and user comfort. In particular embodiments, the sensor electrodes 120 may be formed of an alloy of tin and nickel with a shiny or mirror surface finish. Moreover, in an embodiment, the sensor electrodes 120 may be constructed of a hydrophobic material or a transparent material. For instance, the sensor electrodes 120 may be constructed of glass, sapphire, ceramic, and/or the like with coatings (e.g., for hydrophobicity, wear resistance, and/or the like).

In some embodiments, the wearable computing device 100 may also include at least one additional biometric sensor electrode in addition to the impedance sensor electrodes 120. In such embodiments, the additional biometric sensor electrode may include one or more temperature sensors (such as an ambient temperature sensor or a skin temperature sensor), a humidity sensor, a pressure sensor, a microphone, an optical sensor (e.g., such as a PPG sensor), and/or the like. Thus, the one or more optical sensors may be configured to provide information about heart rate variability (HRV), blood oxygen saturation (SpO2) levels, and the like.

For example, in such embodiments, the PPG sensor(s) described herein may include one or more emitters (e.g., light-emitting diodes (LEDs)) and one or more detectors (e.g., photodiodes). Thus, in an embodiment, the LEDs emit controlled pulses of light and capture the returned light using the photodiodes and the emitted photons reflect off skin, tissue, bones and blood. More specifically, in an embodiment, the PPG sensors described herein may include emitted light wavelengths (e.g., green (~528 nm), red (~660 nm), and infrared (~940 nm)) to measure changes in blood oxygen levels, relying on the differential absorption properties of oxygenated and deoxygenated hemoglobin. In further embodiments, sensors having additional wavelengths, such as near-infrared spectroscopy (NIR) and/or ultraviolet (UV) may also be utilized.

Further, in an embodiment, an Analog Front End (AFE) controls the LEDs and converts the analog current received by the photodiodes into a digital PPG signal. As will be described further herein, changes in the PPG signal associated with oxygenation status manifest typically at red and infrared wavelengths due to differential absorption at red wavelengths of oxygenated and deoxygenated hemoglobin; whereas infrared wavelengths are near the isosbestic point of these two hemoglobin states, providing a meaningful reference against which red wavelength measurements may be compared.

For instance, in the schematic diagram of the system 150 shown in FIG. 2, the wearable computing device 100 includes an optical sensor 122 including one or more detectors 124 for detecting light and one or more light sources or emitters 126 (e.g., LEDs) for emitting light. The detectors 124 and emitters 126 may be arranged within the housing 102 and at least partially exposed through the lower housing cover 114 of the housing 102. The detectors 124 may be used alone and be used to detect ambient light (light not emitted from the wearable computing device 100) or may be used in combination with the emitters 126 such that the detectors 124 detect both ambient light and light from the emitters 126. Use of emitters 126 may particularly be useful in dark environments, where there is little ambient light. The data generated by the sensor(s) 122 may be used to determine a HR estimate or other physiological metrics and/or to determine a distance of the lower housing cover 114 of the wearable computing device 100 from an object, such as the user's forearm 104 (FIG. 1A).

As further shown in FIG. 2, the system 150 may also include at least one controller 152. In an embodiment, the controller(s) 152 may be a central processing unit (CPU) or graphics processing unit (GPU) for executing instructions that can be stored in a memory device(s) 154, such as flash memory, read-only memory (ROM), random access memory (RAM), or dynamic random access memory (DRAM), among other such options, or other non-transitory digital data storage. Further, the memory device(s) 154 may include a control program including sequences of instructions which, when loaded from the memory device 154 and executed using the controller(s) 152, cause the controller(s) 152 to perform the functions that are described herein. As would be apparent to one of ordinary skill in the art, the system 150 can include many types of memory, data storage, or computer-readable media, such as data storage for program instructions for execution by the controller or any suitable processor. The same or separate storage can be used for images or data, a removable memory can be available for sharing information with other devices, and any number of communication approaches can be available for sharing with other devices.

The system 150 also includes one or more power components 156, such as may include a battery operable to be recharged through conventional plug-in approaches, or through other approaches such as capacitive charging through proximity with a power mat or other such device.

In addition, as shown, the system 150 includes any suitable user interface elements in communication with the controller 152, such as the display 116 of the wearable computing device 100. The display 116 may be any suitable display type, such as a touch screen, organic light emitting diode (OLED), liquid crystal display (LCD), and/or the like. In further embodiments, the system 150 can also include at least one additional input/output (I/O) device 158 configured to allow the controller 152 to receive conventional inputs from a user. These conventional inputs can include, for example, a push button (e.g., button 128 in FIG. 1B), touch pad, touch screen, wheel, joystick, keyboard, mouse, keypad, and/or any other such device or element whereby a user can input a command to the system 150. In another embodiment, the I/O device(s) 158 may be connected by a wireless infrared or Bluetooth or other link as well in some embodiments. In some embodiments, the I/O device(s) 158 may additionally, or alternatively, include a microphone or other audio capture element that accepts voice or other audio commands. For example, in particular embodiments, the system 150 may not include any buttons, but might be controlled only through a combination of visual and audio commands, such that a user can control the wearable computing device 100 without having to be in contact therewith. In certain embodiments, the I/O elements 158 may also include one or more of the sensor electrodes 120 described herein, optical sensor(s) (e.g.,

sensor(s) 122), barometric sensors (e.g., altimeter, etc.), and the like.

The system 150 may also include one or more wireless components 160 operable to allow the controller 152 to communicate with one or more electronic devices within a communication range of the particular wireless channel. The wireless channel can be any appropriate channel used to enable devices to communicate wirelessly, such as Bluetooth, cellular, near-field communication (NFC), Ultra-Wideband (UWB), or Wi-Fi channels. It should be understood that the system 150 can have one or more conventional wired communications connections as known in the art.

Still referring to FIG. 2, the sensor(s) 122, may be coupled to the controller 152 directly or indirectly using driver circuitry 162 by which the controller 152 may drive the emitter(s) 126 and obtain signals from the detector(s) 124.

Moreover, the system 150 may include one or more internal motion sensors 164 (e.g., accelerometers, gyroscopes, and/or the like) inside the housing 102 and configured to generate motion data indicative of movement of the wearable computing device 100, with the motion sensors 164 being in communication with the controller 152.

A host computer 168 can communicate with the wireless networking components 160 via one or more networks 166, which may include one or more local area networks, wide area networks, UWB, and/or internetworks using any of terrestrial or satellite links. In some embodiments, the host computer 168 executes control programs and/or application programs that are configured to perform some of the functions described herein.

In some embodiments, the system 150 may include at least one imaging element, such as one or more cameras that are able to capture images of the surrounding environment and that are able to image a user, people, or objects in the vicinity of the device. The imaging element can include any appropriate technology, such as a charge-coupled device (CCD) image capture element having a sufficient resolution, focal range, and viewable area to capture an image of the user when the user is operating the device. Further image capture elements may also include depth sensors. Methods for capturing images using a camera element with a computing device are well known in the art and will not be discussed herein in detail. It should be understood that image capture can be performed using a single image, multiple images, periodic imaging, continuous image capturing, image streaming, etc. Further, the system 150 can include the ability to start and/or stop image capture, such as when receiving a command from a user, application, or other device.

In general, the user might have a wearable computing device, such as a smartwatch or fitness tracker (e.g., the wearable computing device 100), which the user would like to be able to communicate with other devices, such as a smartphone, a tablet computer, and/or the like. Applications may allow communication between multiple devices and a wearable computing device to enable a user to obtain information from the wearable computing device. For example, data captured using a sensor of the wearable computing device 100 may be communicated to the smartphone and/or the tablet computer using an application installed on the smartphone and/or the tablet computer. The user may also want the wearable computing device 100 to be able to communicate with a service provider, such as with the host computer 168 of a service provider, or other such entity, that is able to obtain and process data from the wearable computing device 100 and provide functionality that may not otherwise be available on the wearable computing device 100 or applications installed on the other devices. In addition, as shown, the wearable computing device 100 may be able to communicate with the service provider (e.g., the host computer 168 of the service provider) through at least one network (e.g., the network 166), such as the Internet or a cellular network, or may communicate over a wireless connection such as Bluetooth® to the other device(s) (e.g., the smartphone and/or the tablet computer), where the other device(s) then communicate with the service provider over the at least one network. There may be a number of other types of, or reasons for, communications in various embodiments.

In addition to being able to communicate, a user may also want the devices to be able to communicate in a number of ways or with certain aspects. For example, the user may want communications between the devices to be secure, particularly where the data may include personal health data or other such communications. The device or application providers may also be required to secure this information in at least some situations. The user may want the devices to be able to communicate with each other concurrently, rather than sequentially. This may be particularly true where pairing may be required, as the user may prefer that each device be paired at most once, such that no manual pairing is required. The user may also desire the communications to be as standards-based as possible, not only so that little manual intervention is required on the part of the user but also so that the devices can communicate with as many other types of devices as possible, which is often not the case for various proprietary formats. A user may thus desire to be able to walk in a room with one device and have such device automatically communicate with another target device with little to no effort on the part of the user. In various conventional approaches, a device will utilize a communication technology such as Wi-Fi to communicate with other devices using wireless local area networking (WLAN). Smaller or lower capacity devices, such as many Internet of Things (IoT) devices, instead utilize a communication technology such as Bluetooth®, and in particular Bluetooth Low Energy (BLE) which has very low power consumption.

In further embodiments, data may be captured, processed, and displayed in a number of different ways. For example, data may be captured using sensors on the wearable computing device 100, but due to limited resources on the wearable computing device 100, the data may be transferred to the smartphone, the tablet computer, and/or the service provider (or a cloud resource) for processing, and results of that processing may then be presented back to that user on the wearable computing device 100, smartphone, the tablet computer and/or another such device associated with that user. In at least some embodiments, a user may also be able to provide input such as health data using an interface on any of these devices, which can then be considered when making that determination.

As previously mentioned, unanticipated drops in oxygenation that do not recover represent time sensitive emergencies and can arise from a number of causes. In certain instances, early detection and rapid intervention can prevent death from these events. For example, such impaired oxygenation events usually begin with slower motion rates with a slight decrease in the blood O2 saturation level. The continuous lack of oxygenation intake further reduces the O2 saturation level fairly rapidly (less than half an hour) with further reduced motion capacity, until the O2 saturation drops to a dangerous level (usually below 83%) where the low O2 saturation may affect brain function if it persists for longer than 5-10 minutes.

Thus, the present disclosure is directed to an oxygen emergency detection system for a wearable computing device configured to detect such life-threatening O2 saturation drops within this latency, in a reasonably high sensitivity and sufficiently low specificity manner that would not affect daily usage for healthy users. For example, the present disclosure capitalizes on the distinct physiological characteristics of oxygen emergency events to enhance sensitivity and specificity. In such embodiments, these characteristics include continuous and non-recovering low SpO2, absence of active motion, and a rapid relative drop in SpO2. In addition, the impact on battery life and memory consumption significantly influences the feasibility of on-device implementation for detected events. Thus, the present disclosure employs a multi-phase approach that executes complex models only when deemed necessary. This approach ensures minimal battery and memory consumption during most periods, making the system usable on a mass market consumer wearable device. Additionally, the present disclosure utilizes compact models with, e.g., linear algorithms and implements duty cycles when appropriate.

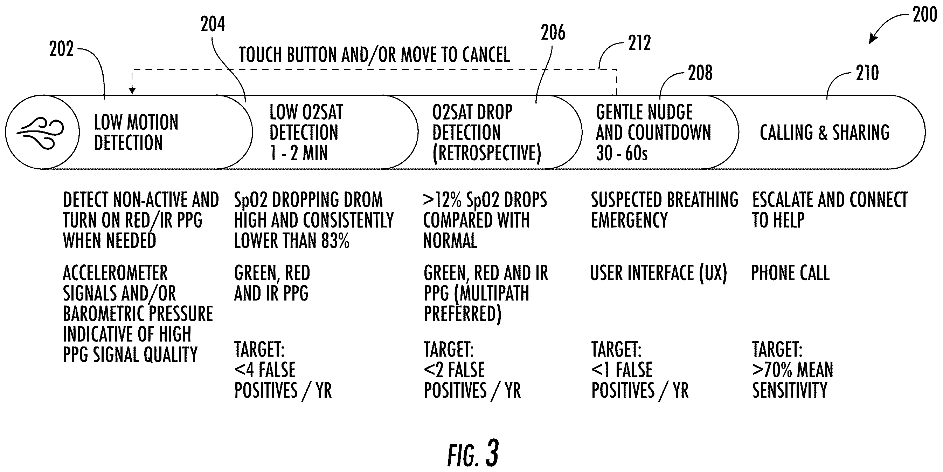

Accordingly, in an embodiment, the oxygen emergency detection system of the present disclosure includes several phases, where a latter phase runs only when its former phase passes. For example, as shown in FIG. 3, a schematic diagram of an embodiment of phase progression 200 of the oxygen emergency detection system according to the present disclosure is illustrated.

In particular, as shown, the phase progression of the oxygen emergency detection system includes an always-on low motion detection phase 202 as a prerequisite for an oxygen emergency event is for the user to be in a predominantly still or slow-moving state. Thus, during the low motion detection phase 202, in an embodiment, the oxygen emergency detection system is configured to detect whether a user is non-active (e.g., still, non-moving, or slow-moving). For example, in an embodiment, the oxygen emergency detection system is configured to employ accelerometer signals from one or more accelerometers of the wearable computing device 100 to ascertain whether the user is actively engaged in movement (for example, walking or exercising), which are not consistent with emergent, low O2 state. In an embodiment, the accelerometer(s) described herein may include, for example, a triaxial accelerometer. This also serves to minimize unnecessary inferences during instances when PPG signals are anticipated to be noisy.

Furthermore, in an embodiment, previous studies have shown that opioid consumption severely decreases the frequency of limb movement. To this end, in an embodiment, the low motion detection phase 202 may apply a high pass filter to the acceleration data using exponential decaying techniques with alpha=0.85. Thus, this action is configured to filter out slow motion such as breathing and unconscious movement. After applying this high pass filter to each of the three axes of the accelerometer data, the low motion detection phase 202 is configured to calculate peak-to-peak to each of the axes for a particular time frame (e.g., such as a couple of seconds) and sum the three components together. Thus, the low motion detection phase 202 is configured to consider the user to be in a low motion state if the peak-to-peak sum is below a certain threshold, such as about 0.3g. If the user is in a low motion state, the phase progression 200 of the oxygen emergency detection system is configured to proceed to the next phase.

More specifically, as shown at (204), the oxygen emergency detection system is configured to employ low O2Sat detection. In this subsequent phase, the oxygen emergency detection system is configured to employ a compact ML model that forecasts whether the user has a consistent low SpO2 reading over a specified duration. Concurrently, the oxygen emergency detection system is also configured to detect intervals of high (that is, normal) SpO2 levels and store the corresponding raw PPG signals so as to serve as a reliable baseline for a relative change.

More specifically, in an embodiment, the low O2Sat detection model 204 encompasses a substantial portion of daytime use, whenever the low motion detection phase 202 is successfully completed. As a result, the low O2Sat detection model 204 is economical to operate and supports duty cycling, such as running once per minute, to minimize the utilization of red/IR LEDs. As an initial gateway employing PPG signals toward potential emergency detection, this phase is configured to prioritize sensitivity over specificity, where the next phase could collectively help.

In an embodiment, the low O2Sat detection model 204 may utilize an existing SpO2 estimation model of the wearable computing device 100. In such embodiments, it is possible to leverage the existing SpO2 estimation model for the purpose of estimating high or low SpO2 states. Notably, the state-of-the-art SpO2 ML model accomplishes a high level of overall SpO2% accuracy, as verified through comprehensive evaluation. In such embodiments, utilizing the existing SpO2 estimation model provides a mature training and evaluation pipeline, has more interpretable results, post-processing possibilities are based on estimated SpO2% distributions, and has potential memory conservation through sharing a common serving model.

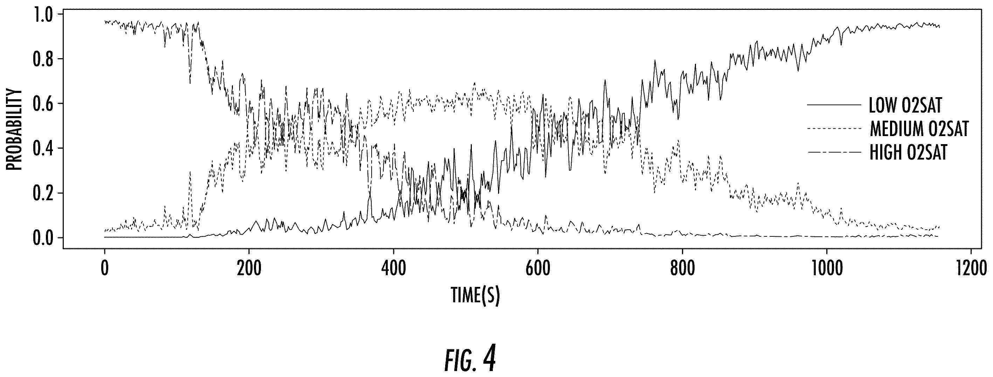

In another embodiment, the low O2Sat detection model 204 may utilize a specialized SpO2 classification model with three distinct output classes: high, medium, and low (also referred to herein as a tri-class ML model). In such embodiments, the thresholds for each class can be tailored to strike a balance between latency, sensitivity, and specificity. In such embodiments, utilizing the specialized SpO2 classification model provides for a smaller model with potentially shorter receptive fields, thereby enabling more frequent duty cycling, increases flexibility for the integration of novel preprocessing algorithms that may further reduce the computational costs, and provides further customization capabilities to allow for fine-tuning possibilities without compromising the accuracy of another application. As shown particularly in FIG. 4, an example of a tri-class ML model with tri-class probabilities (i.e., low, medium, and high O2Sat values) along time for an O2Sat drop session is provided, where the oxygenation saturation of individual drops continuously for about 20 minutes from more than 95% to less than 80% without recovery is illustrated.

The low O2Sat detection model 204 is also configured to implement various preprocessing methods. For example, the green, red and IR channel data streams of the PPG sensors described herein may be first filtered by motion and LED current change and then filtered, such as via band-pass filters, to extract an alternating current (AC) component and a direct current (DC) component. In certain embodiments, the band-pass filters may be 2nd- or 3rd-order Infinite Impulse Response (IIR) filters, such as Butterworth filters, with 0.4-0.5Hz cut-off frequency, allowing preserving respiratory traces in the DC component while keeping the frequency response in the heart rate range flat in the AC component. Moreover, in an embodiment, the band-pass filters may be implemented such that the filters reset their states at gaps from motion or LED current change. During reset, the internal states are initialized as if an infinite number of samples identical to the incoming sample had been fed. This is to minimize the oscillation from signal discontinuity.

In further embodiments, the band-passed signals may then be combined through a perfusion normalization filter borrowed from the existing SpO2 ML model preprocessor. In particular embodiments, Equation (1) below can be used to calculate a ratio-of-ratios, in which a ratio of heart-beat modulated reflection (e.g., AC component) to the average reflection (e.g., DC component) is calculated for both red and infrared wavelengths:

$$\frac{AC}{DC} * \frac{DC_{ref}}{mean(AC_{ref})} \qquad \text{Equation (1)}$$

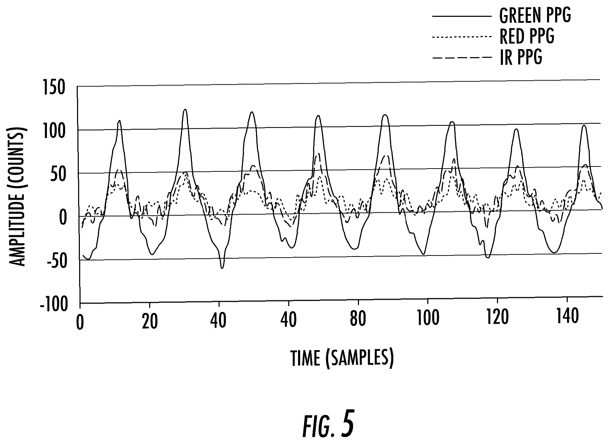

where a reference channel (e.g., green) is used to normalize the output to approximately around 1. Thus, in such embodiments, the ratio-of-ratios calculation of Equation (1), using AC/DC components of reflected light, provides a measure of SpO2. As an example, FIG. 5 illustrates how the reflected light for green, red, and infrared PPG signals modulate with pulsatility and how all three wavelengths have both the AC and the DC component useful to the computation of a ratio-of-ratios. Moreover, as shown, FIG. 5 illustrates that the reflected light for the green, red and infrared PPG signals change with every heartbeat.

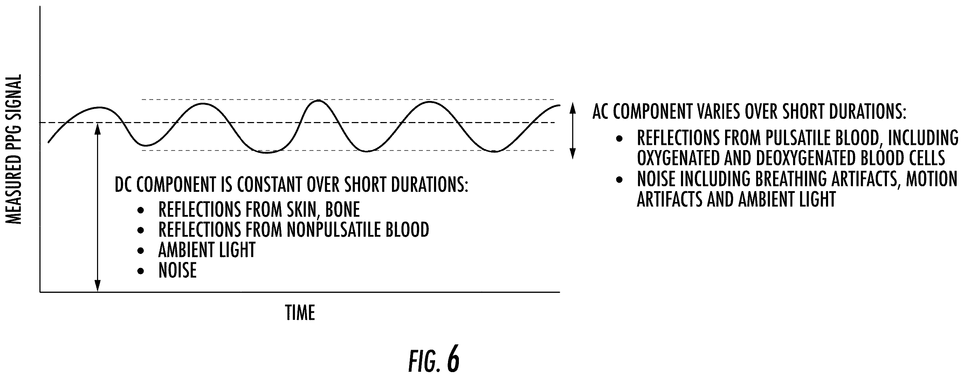

Accordingly, in an embodiment, this phase of the algorithm is designed to detect substantial SpO2 drops and persistent hypoxia states using three PPG channels using green, red, and infrared lights (e.g., with a wavelength of 528 nm, 660 nm, and 940 nm, respectively). Oximetry measures blood oxygen levels by analyzing the differing reflection of red and infrared light from oxygenated and deoxygenated blood. Thus, as shown in FIG. 5, each heartbeat modulates the reflected light. SpO2 can be determined by correlating it with the "ratio-of-ratios", which is the ratio of heartbeat-modulated reflection (e.g., the AC component) to average reflection (e.g., the DC component) for both red and infrared wavelengths. Using the principle above, and as shown in FIG. 6, the algorithm described herein is configured to extract features of the measured PPG signal(s) related to the AC and DC components of PPG signals. More specifically, in an embodiment, a convolutional neural network (CNN) consumes these features to classify such signals into normoxia ($SpO2 \geq 92\%$), transition ($83\% \leq SpO2 < 92\%$), and hypoxia ($SpO2 < 83\%$). Additionally, a state machine is configured to confirm rapid SpO2 drops and persistent hypoxia, provided the PPG signal passes quality checks and is deemed valid as further described herein.

In additional embodiments, the preprocessed signals are fed into either the existing SpO2 estimation ML model or the tri-class ML model. In such embodiments, if using the SpO2 estimation ML model, the outcome for each time step may include a percentage estimation alongside a standard deviation estimation. For example, in an embodiment, the SpO2 estimation can be preprocessed into three bins (e.g., <83%, 83-93%, >93%). In such embodiments, if standard deviation goes above 8, the corresponding time step can be marked as unavailable since the estimate may be unreliable.

In contrast, if using the tri-class ML model, the outcome for each time step is the probability of falling into each of the three bins. In such embodiments, as an example, if the difference between the maximum probability and the minimum probability exceeds a certain value, such as 0.2, the corresponding time step can be marked as unavailable.

Meanwhile, the low O2Sat detection model 204 may also maintain a buffered data set of a predetermined size, such as 3, that stores the latest raw PPG signal segments that correspond to the receptive field of the highest SpO2 estimation (if using the SpO2 estimation ML model), or the highest high class probability (if using the tri-class model).

These raw segments can thus be used for comparison in a later phase as described herein. Thus, in an embodiment, the low O2Sat detection model 204 is configured to determine results on each time step as being categorized as one of the following: unavailable (due to not passing the low motion detection phase, or an unreliable estimation result), low O2Sat (<83%), medium O2Sat (83%-93%), or high O2Sat (>93%). Accordingly, in an embodiment, as a particular example, the oxygen emergency detection system only advances to the subsequent phase if and only if: at least 10 seconds of high O2Sat within the past 120 seconds to 30 minutes, at least 80% are low O2Sat within the past 60 seconds, and at least 10 seconds medium O2Sat between the latest 10 seconds high O2Sat and 60 seconds before the current time step.

Accordingly, and referring back to FIG. 3, as shown at (206), the oxygen emergency detection system is further configured to implement the next phase, i.e., an O2Sat drop detection model 206 upon satisfaction of phases 202 and 204. In certain embodiments, the O2Sat drop detection model 206 may be optional.

In an embodiment, the O2Sat drop detection model 206 includes employing a relative change detection model that juxtaposes the current PPG with a purportedly low SpO2 against the most recently stored PPG signals with a purportedly normal SpO2. In such embodiments, the O2Sat drop detection model 206 may subsequently estimate whether the underlying SpO2 differential is sufficiently substantial and persistent. Persistence can be important as some relative drops in SpO2 recover quickly and do not represent an emergency (e.g., undiagnosed sleep apnea).

Accordingly, the O2Sat drop detection model 206 is configured to ascertain a suspected oxygen emergency by discerning a significant underlying SpO2 reduction from the same user within a specified time frame. Thus, in an embodiment, the O2Sat drop detection model 206 is configured to minimize inter-population biases and employ multiple optical paths. Accordingly, in an embodiment, this phase would not be triggered frequently (such as under once per day).

Moreover, during this phase, the buffered data set populated by the low O2Sat detection model 204 can be utilized. For example, in an embodiment, the buffered data set contains the most recent raw PPG signals with an underlying normal/high SpO2. Moreover, in an embodiment, this phase is initiated only when the preceding phase has been successfully completed and passed, indicating a suspicion of a low underlying SpO2 for the user. Consequently, a significant underlying SpO2 difference is expected when comparing the current PPG signals with those stored in the buffered data set.

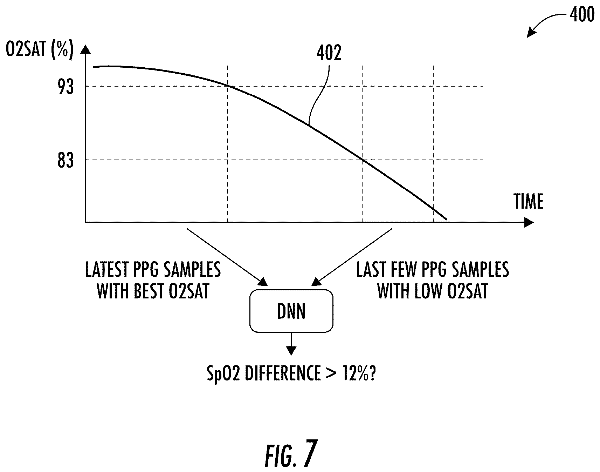

Based on this hypothesis, the O2Sat drop detection model 206 is configured to employ an additional ML model to forecast whether the underlying SpO2 difference between two distinct PPG segments is below or above a specific percentage threshold (e.g., which may be set at 12%). For example, as shown in FIG. 7, a graph 400 of O2Sat as a percentage (%) versus time is illustrated. As indicated by curve 402, the O2Sat of the user starts above 93% and drops over the illustrated time period to below 83%. In such embodiments, the model may utilize two sets of PPG channels (e.g., red, green, and IR) as inputs and may generate a binary classification as an output. As shown particularly in FIG. 7, a deep neural network (or any suitable classifier) may receive the latest PPG samples with the best (i.e., normal) O2Sat values and the last few PPG samples with low O2Sat values to determine the SpO2 difference.

Given the infrequent execution of this phase, the O2Sat drop detection model 206 has the capacity to compare the current PPG segment with each previous PPG segment in the buffered data set. Additionally, the O2Sat drop detection model 206 can execute multiple paths for each time step. Accordingly, in an embodiment, the O2Sat drop detection model 206 may calculate predictions per time step, e.g., by multiplying the buffered data set size (e.g., 3) by the number of paths (e.g., 4), which in the illustrated embodiment is equal to 12 predictions per time step. In such embodiments, if a majority of these predictions passes for three consecutive time steps, the oxygen emergency detection system moves to the next phase.

For example, referring still to FIG. 3, as shown at (208), in the event that all three phases are sequentially passed through (i.e., 202, 204, and 206), the oxygen emergency detection system is configured to implement an alert (such as a gentle nudge, haptic feedback, an alarm, etc.) and countdown to ensure that an emergency is actually taking place. In such embodiments, the oxygen emergency detection system has determined that a suspected oxygen emergency is occurring and may request a user response within e.g., 30-60 seconds. For example, as shown at (212), if an oxygen emergency is not occurring, the user can engage the wearable computing device 100, e.g., via the screen, a button, a tap, and/or movement to cancel further action. For example, in an embodiment, the wearable computing device 100 can include a motion cancellation feature which allows the user to deactivate the oxygen emergency detection via movement, such as the user moving his arm. In such embodiments, further action is cancelled simply by user movement, rather than requiring the user to engage a screen, button, etc. of the wearable computing device 100.

If, however, the user does not respond, as shown at (210), the oxygen emergency detection system is configured to escalate the situation and connect the user to help, e.g., by placing an emergency call (to an emergency telephone number, such as 911 or 112, or close contacts) via the user's connected compatible hardware, such as a smartphone or smartwatch (which may be the wearable computing device 100 itself) using broadband or cellular phone technology. In an embodiment, for example, if no response is received by the user, the wearable computing device 100 may sound an audible notification and implement a countdown to call an emergency telephone number, such as 911 or 112.

Thus, by implementing the multi-phase approach, the number of false positives is decreased with each phase, thereby increasing sensitivity of the system. In an embodiment, all phases of the oxygen emergency detection system may be implemented quickly, for example, in about five (5) minutes or less. Furthermore, in an embodiment, to avoid errant emergency calls, the oxygen emergency detection system may include one or more safeguards, such as identifying periods of potential impaired oxygenation state by both persistently low absolute oxygen saturation levels and a large relative drop in oxygen saturation levels and issuing audio, visual, and haptic alerts to the user to encourage the user to de-escalate the emergency response.

In certain embodiments, the O2Sat drop detection model 206 may occur before the low O2Sat detection model 204, as the drop in O2Sat precedes a threshold violation. However, in other embodiments, it may be beneficial to maintain the O2Sat drop detection model 206 after the low O2Sat detection model 204 to allow for easier design and training of the O2Sat drop detection model 206. For example, in an embodiment, the O2Sat drop detection 206 may require an extensive receptive field to accurately capture substantial relative change, resulting in increased memory requirements for buffering raw and intermediate data, whereas the low O2Sat detection model 204 requires a smaller receptive field and model size. Thus, by implementing the O2Sat drop detection model 206 after the low O2Sat detection model 204, the complexity of the ML model required for the O2Sat drop detection model 206 is reduced. Additionally, by incorporating high O2Sat detection alongside low O2Sat detection, necessary receptive fields could be provided for the O2Sat drop detection model 206.

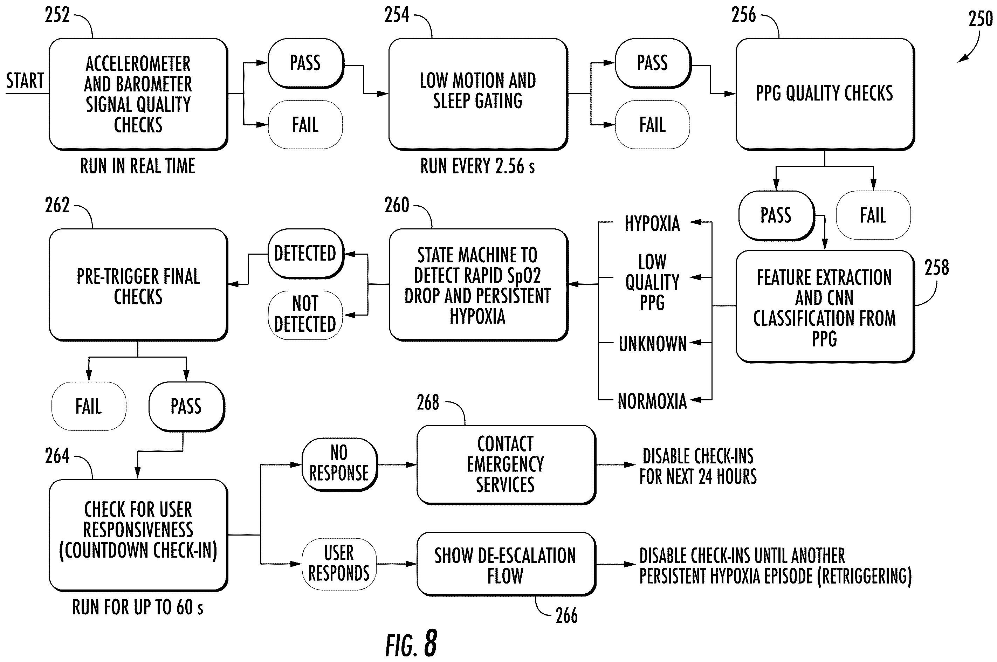

Referring now to FIG. 8, a flow diagram 250 of the various, detailed algorithmic stages of the oxygen emergency detection system described herein is illustrated. In particular, as shown at (252), the accelerometer sensor can be used by the algorithm to identify periods of low motion, given that low motion is a proxy for PPG signal quality. The input accelerometer signal quality checks are run before any accelerometer data are processed by the algorithm, ensuring that the data are not all identical, are not too variable, are not saturated, and are sampled with sufficient resolution.

Further, in an embodiment, a barometer can be used by the algorithm for signal quality checks, as the barometer measures the atmospheric pressure to which the wearable computing device 100 is exposed. Thus, this pressure is indicative of the approximate elevation/altitude of the user. This is beneficial because blood and tissue oxygen saturation levels are reduced at higher elevations due to the lower partial pressure of oxygen in the atmosphere at increased elevation. In such embodiments, the barometer signal quality checks ensure that the PPG data analyzed by the algorithm have sufficient quality. Further, the barometer checks ensure that the barometer data are not too variable, exceed a threshold, and are sampled at a sufficient resolution. If the quality checks are not satisfied, the algorithm ends, as shown at FAIL. If both quality checks are satisfied (e.g., PASS in FIG. 8), the algorithm proceeds to (254), which involves detecting low motion and sleep gating.

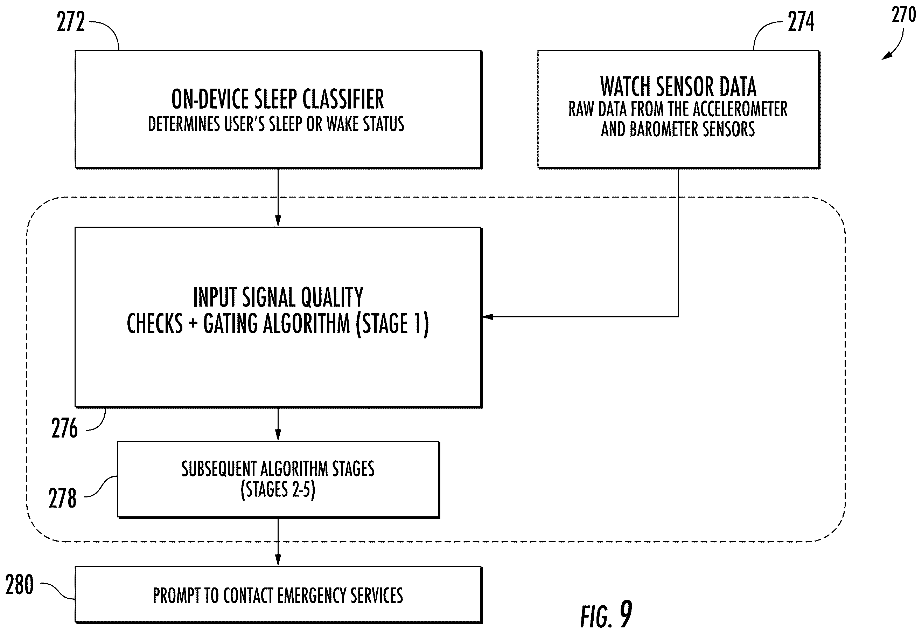

Impaired oxygenation is presumed to occur when a user is largely motionless or moving slowly. Detecting this non-motion state involves computing the peak-to-peak value from high-pass filtered accelerometer signals within a sliding window. Further, and referring to FIG. 9, a flow diagram of an embodiment of a sleep gating algorithm 270 of the oxygen emergency detection system described herein is illustrated. In particular, as shown, the sleep gating algorithm 270 processes data from an on-device sleep classifier 274 and/or watch sensor data 274 to identify periods of persistent sleep. In an embodiment, the data processing occurs only if the input signal quality checks are met. These input quality checks verify that one or more "asleep" or "awake" classifications are present and that an excessive number of asleep to awake or awake to asleep transitions are not present. If the input signal quality checks are met, the asleep/awake states are analyzed by a gating algorithm 276. The gating algorithm 276 analyzes the asleep/awake state data to determine if the user has been persistently asleep in the recent past, and if so, to disable running the subsequent algorithm phases (e.g., phases 256 and beyond of FIG. 8). However, if the user has not been persistently asleep, the algorithm proceeds to subsequent algorithm stages (e.g., as shown at 278) and prompts to contact emergency services (e.g., as shown at 280), as further explained herein below.

As defined by the algorithm, the term "persistently" asleep is carefully chosen to minimize the risk of false negatives, given that users who experience breathing emergencies may inadvertently appear to be "asleep" given their low motion state. As such, in an embodiment, the algorithm generally defines "persistently asleep in the recent past" as users who have been asleep for more than about two hours in the past 2.5 hours. This definition corresponds to having the vast majority (≥80%) of the asleep/awake states in the recent past indicating that the user is asleep (in order to address scenarios such as the user getting up to use the bathroom or get water in the middle of the night) while also accommodating the various causes of breathing emergencies described herein. In an embodiment, the sleep gating algorithm 276 can be implemented by subscribing to the wearable computing device 100 a bedtime mode state change, and counting the duration of sleeping (i.e., bed-time mode on) episodes within a rolling window of 2.5 hours. If it is less than hours (e.g., PASS in FIG. 8), the algorithm can proceed to (256) as shown in FIG. 8. If more than hours, the algorithm ends, as shown at FAIL.

More specifically, as shown at (256), the oxygen emergency detection system is configured to perform PPG quality checks. In particular, the PPG sensor(s) described herein can be used by the algorithm to identify periods of hypoxia that had normoxia in the recent past. The input PPG signal quality checks are run before any PPG data are processed by the algorithm, ensuring that the data are not all identical, are not saturated, and are sampled with sufficient temporal resolution. An additional algorithm verifies that the input sensor data has sufficiently high cross-correlation indicating similarity across channels.

As shown at (258), the algorithm continues to feature extraction related to the AC and DC components of PPG signals and CNN classification of the PPG signals. More specifically, in an embodiment, a CNN is configured to receive these features as inputs to classify such signals into the following categories: normoxia (SpO2 ≥ 92%), transition (83% ≤ SpO2 < 92%), hypoxia (SpO2 < 83%), and unknown. Thus, as shown at (260), the algorithm continues to a state machine configured to detect rapid SpO2 drops and persistent hypoxia.

If rapid SpO2 drops and persistent hypoxia are not detected, the algorithm ends. If rapid SpO2 drops and persistent hypoxia are detected, the algorithm proceeds to (262) to retrospectively perform a few additional PPG signal quality checks to ensure that the hypoxia predictions are not mistakenly derived from bad quality signals. For example, in an embodiment, the algorithm is configured to quantify the periodicity of the past e.g., 10 seconds of PPG signal data via autocorrection and identify a lack of periodicity indicative of low signal quality. The autocorrelation of a signal quantifies how correlated a signal is with a time-delayed version of itself. High values indicate strong periodicity (e.g., a sinusoid) and low values indicate weak periodicity (e.g., a random signal). The short duration of the analyzed window (e.g., 10 seconds) ensures that there is minimal periodicity present from respiration while ensuring that there are multiple cycles of periodicity from pulsatility (should pulsatility be present). In an embodiment, autocorrelation checks can be performed on the AC component of the green, red, and infrared PPG channels, respectively. More specifically, in an embodiment, the check passes if two out of the three channels have an autocorrelation value below a threshold (such as 0.2 in normalized units).

Still another quality check may include a breathing interference check. Occasionally, strong breathing patterns may infiltrate filtered PPG signals, thereby causing substantial interference and hindering the accurate extraction of pulse characteristics. This can result in inaccurate SpO2 predictions. To mitigate this, the algorithm can perform one or more breathing interference checks using signal-to-noise (SNR) estimates. More specifically, the algorithm can perform Fast Fourier Transform (FFT) operation over a certain time period, such as the past 20 seconds of filtered PPG signal data and obtain the total energy falling within [9 (beats per minute (BPM), 39 BPM] (the noise) and [50 BPM, 150 BPM] (the signal), respectively. In such embodiments, the check passes if the ratio between them is below a certain threshold (e.g., 0.4 for red and infrared PPG channels, and 0.3 for the green PPG channel).

A further quality check may also include checking the past few hypoxia probabilities from the ML model to ensure that the algorithm is confident in the predictions. In such embodiments, the algorithm is configured to collect recent hypoxic class output values (such as the last five) from the ML model. In an embodiment, this check passes if two out of the five are more than 0.6.

Yet another quality check may include test-time augmentation, which is a technique where during inference, the algorithm can perform a few different augmented versions from the original signal to improve accuracy. The general idea of this optional check is to synthesize another signal from the existing signal and run the ML inference again to validate the output consensus. In such embodiments, signal reversal can be an effective signal synthesization method. To implement this, in an embodiment, the algorithm is configured to store a certain time frame of sensor signals (such as the last 15 seconds) and before a potential alert, the model buffer can be cleared and rerun using the recently stored buffer reversely from back to the front. The algorithm would only trigger when the rerun outputs a hypoxia probability greater than a certain threshold (such as 0.3).

If one or more of the quality checks fail (as shown at FAIL in FIG. 8), the algorithm ends. If all quality checks pass (e.g., as shown at PASS in FIG. 8), the algorithm proceeds to (264). In particular, as shown at (264), this phase checks for user responsiveness and escalates to a countdown timer to a call to close contacts or emergency medical services (EMS) (as shown at (268)). At a high level, the feature displays a notification and provides a strong haptic stimulus to the user. The design is driven by the insight that the user may still be conscious during this stage, and they may be alerted by the haptics, and choose to ignore if they are feeling well, or directly choose to place an emergency call if they want help.

In an embodiment, the haptics pattern used in this stage are designed to catch a user's attention and may not be used in other features, so as to stand out to the user. As an example, the haptics pattern may include a series of long and short duration haptics at maximum intensity, e.g., for 30 seconds. As such, the haptics duration is designed to be sufficiently long to minimize the risk of false positives from users who did not actually experience an impaired oxygenation event (e.g., by giving those users adequate opportunity to respond) while being sufficiently short to minimize the time to calling emergency services in users who experience impaired oxygenation events.

Furthermore, in an embodiment, the algorithm is configured to analyze data from the accelerometer sensor to determine if a user responds to the haptic prompt. The design of this portion of the algorithm is broadly similar to the stillness algorithms from the prior stages with one exception: the accelerometer sensor data in all axes are first smoothed over 100 milliseconds to smooth out the haptics pattern. This is because the haptics pattern for this algorithm stage on its own causes the housing 102 of the wearable computing device 100 to move sufficiently to exceed the stillness thresholds from the prior algorithm phases.

If the user responds, as shown at (266), the algorithm shows the de-escalation flow and disables check-ins until another persistent hypoxia episode occurs. If the user does not manually clear the notification by interacting with the wearable computing device 100 nor by showing purposeful motion, as shown at (268), then the algorithm proceeds to contacting emergency services.

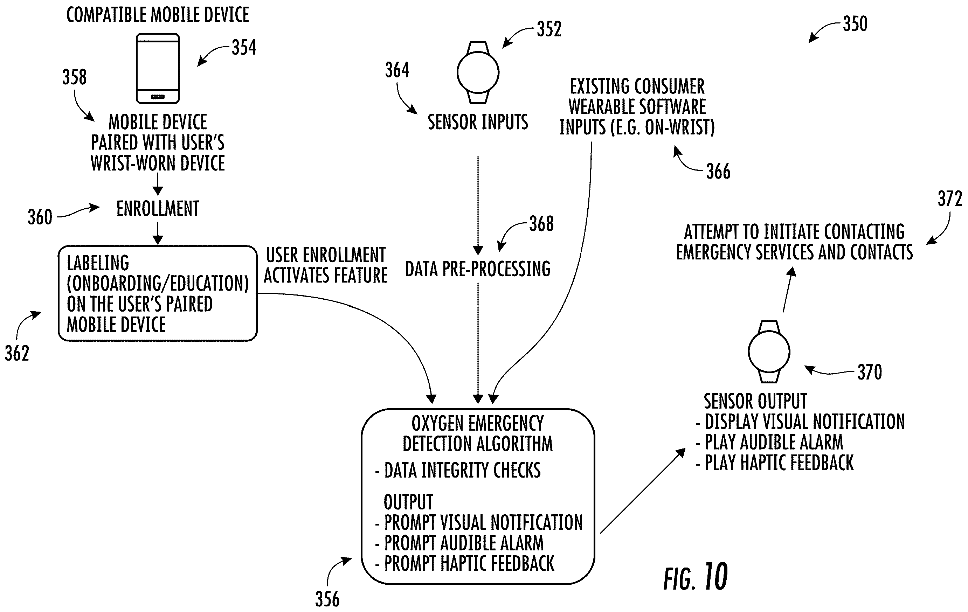

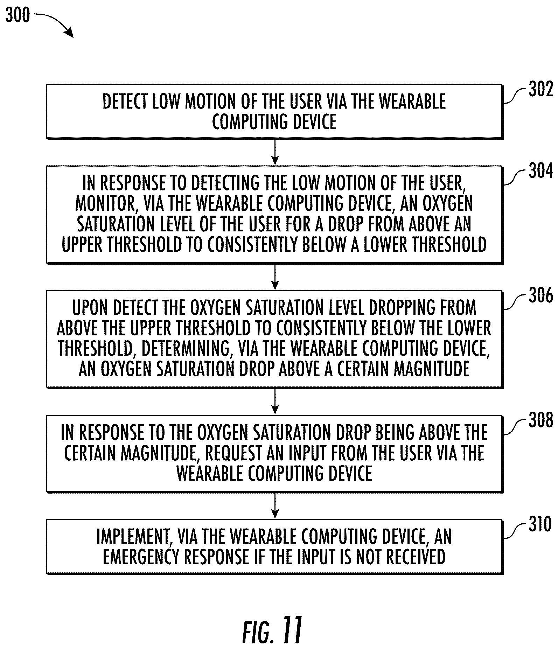

Referring now to FIGS. 10 and 11, a system 350 and method 300 of detecting and responding to an oxygen emergency of a user of a wearable computing device (e.g., wearable computing device 100) are illustrated in accordance with aspects of the present subject matter. In particular, FIG. 10 illustrates a schematic diagram of various components of the system 350 of detecting and responding to an oxygen emergency of a user of a wearable computing device, whereas FIG. 11 illustrates a flow diagram of a method 300 of detecting and responding to an oxygen emergency of a user of a wearable computing device in accordance with aspects of the present subject matter.

More specifically, as shown in FIG. 10, the system 350 may include a wearable computing device 352, such as the wearable computing device 100 described herein, a compatible mobile device 354, such as a smartphone, and an oxygen emergency detection module 356. Thus, as shown at 358, the compatible mobile device 354 can be paired with the user's wearable computing device 352. Further, as shown at 360, the user can opt to enroll in the oxygen emergency detection feature, which activates the oxygen emergency detection module 356. As shown at 362, upon opting in, the compatible mobile device 354 and/or the wearable computing device 352 can provide education and/or onboarding information to the user, such as what the feature is and how it works, as well as verifying eligibility for the feature. The user may also be allowed to confirm emergency contacts.

Once activated, the wearable computing device 352 collects sensor data 364 (e.g., PPG and accelerometer data as described herein) as well as existing consumer wearable software inputs 366 (e.g., on-wrist detection, etc.) and pre-processes the data, as shown at 368. The oxygen emergency detection system can receive such data and implement one or more data integrity checks before generating one or more outputs (such as prompting a visual notification, prompting an audible alarm, and/or prompting haptic feedback). Thus, as shown at 370, the wearable computing device 352 is configured to display a visual notification to the user, play an audible alarm, and/or play haptic feedback to the user. If no response is received, as shown at 372, the wearable computing device 352 is configured to attempt to initiate contacting emergency services and/or contacts, e.g., via the compatible mobile device 354 and/or the wearable computing device 352.

In general, the method 300 is described herein with reference to the wearable computing device described in FIGS. 1A-10. However, it should be appreciated that the disclosed method 300 may be implemented with any other suitable wearable computing device having any other suitable configurations. In addition, although FIG. 11 depicts steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

As shown at (302), the method 300 may include detecting low motion of the user via the wearable computing device. In response to detecting the low motion of the user, as shown at (304), the method 300 includes monitoring, via the wearable computing device, an oxygen saturation level of the user for a drop from above an upper threshold to consistently below a lower threshold. Upon detecting the oxygen saturation level dropping from above the upper threshold to consistently below the lower threshold, as shown at (306), the method 300 includes determining, via the wearable computing device, an oxygen saturation drop above a certain magnitude. In response to the oxygen saturation drop being above the certain magnitude, as shown at (308), the method 300 includes requesting an input from the user via the wearable computing device. As shown at (310), the method 300 includes implementing, via the wearable computing device, an emergency response if the input is not received.

The technology discussed herein makes reference to servers, databases, software applications, and other computer-based systems, as well as actions taken and information sent to and from such systems. The inherent flexibility of computer-based systems allows for a great variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

While the present subject matter has been described in detail with respect to various specific example embodiments thereof, each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations and/or additions to the present subject matter as would be readily apparent to one of ordinary skill in the art. For instance, features illustrated or described as part of one embodiment can be used with another embodiment to yield a still further embodiment. Thus, it is intended that the present disclosure covers such alterations, variations, and equivalents.

**Claims**

**1.** A wearable computing device, comprising:

one or more sensors for detecting low motion of a user of the wearable computing device;
a processor communicatively coupled with the one or more sensors, the processor configured to perform a plurality of operations comprising:

in response to detecting the low motion of the user, monitoring an oxygen saturation level of the user for a drop from above an upper threshold to consistently below a lower threshold;
upon detecting the oxygen saturation level dropping from above the upper threshold to consistently below the lower threshold, determining an oxygen saturation drop above a certain magnitude;
in response to the oxygen saturation drop being above the certain magnitude, requesting an input from the user via the wearable computing device; and
implementing, via the wearable computing device, an emergency response if the input is not received.

2. The wearable computing device of claim 1, wherein the one or more sensors comprise accelerometers.

3. The wearable computing device of claim 1 or 2, wherein monitoring the oxygen saturation level of the user for the drop from above the upper threshold to consistently below the lower threshold further comprises:

monitoring one or more photoplethysmography, PPG, signals to serve as a reliable baseline for a relative change in the oxygen saturation level; and
employing a machine-learned model using the one or more PPG signals that forecasts whether the oxygen saturation level of the user is consistently below the lower threshold over a specified duration while concurrently detecting intervals of the oxygen saturation level being above the upper threshold.

4. The wearable computing device of any one of claims 1 to 3, wherein monitoring the oxygen saturation level of the user for the drop from above the upper threshold to consistently below the lower threshold further comprises:
utilizing one of an existing oxygen saturation estimation machine-learned model of the wearable computing device or a tri-class machine-learned model having high, medium, and low output classes.

5. The wearable computing device of claim 4, wherein monitoring the oxygen saturation level of the user for the drop from above the upper threshold to consistently below the lower threshold further comprises at least one of the following pre-processing steps:

filtering the oxygen saturation level of the user by at least one of motion or LED current change; or
filtering the oxygen saturation level of the user via one or more band-pass filters to extract DC and AC components.

6. The wearable computing device of claim 5, wherein monitoring the oxygen saturation level of the user for the drop from above the upper threshold to consistently below the lower threshold further comprises:

feeding the filtered oxygen saturation level into one of the existing oxygen saturation estimation machine-learned model or the tri-class machine-learned model,
wherein, when fed into the existing oxygen saturation estimation machine-learned model, an outcome for each time step comprises a percentage estimation alongside a standard deviation estimation, and
wherein, when fed into the tri-class machine-learned model, an outcome for each time step is a probability of falling into each of one of three bins representing the high, medium, and low output classes.

7. The wearable computing device of claim 5 or 6, wherein monitoring the oxygen saturation level of the user for the drop from above the upper threshold to consistently below the lower threshold further comprises:
maintaining a buffered data set of a predetermined size that stores one or more recent photoplethysmography, PPG, signals from one or more PPG sensors that correspond to a receptive field of a highest oxygen saturation estimation when using the existing oxygen saturation estimation machine-learned model or a highest high class probability when using the tri-class machine-learned model.

8. The wearable computing device of claim 7, wherein determining the oxygen saturation drop above the certain magnitude further comprises:

employing a relative change detection model that juxtaposes one or more current PPG signals from the wearable computing device with a purportedly low oxygen saturation level against the one or more recent PPG signals with a purportedly normal oxygen saturation level to determine the oxygen saturation drop;

estimating whether the oxygen saturation drop is sufficiently substantial and persistent so as to indicate a suspected oxygen emergency; and

determining the suspected oxygen emergency is occurring by discerning a significant underlying oxygen saturation reduction from the user within a specified time frame.

9. The wearable computing device of claim 8, wherein determining the oxygen saturation drop above the certain magnitude further comprises:

utilizing the buffered data set to determine the oxygen saturation drop, wherein the significant underlying oxygen saturation reduction is expected when comparing the one or more current PPG signals with the one or more recent PPG signals stored in the buffered data set; and

employing an additional ML model to forecast whether the significant underlying oxygen saturation reduction between two distinct PPG signals is below or above a specific percentage threshold.

10. The wearable computing device of claim 8 or 9, wherein requesting the input from the user via the wearable computing device further comprises:

requesting, via the wearable computing device, a response from the user within a certain time frame after determining the suspected oxygen emergency is occurring to ensure that an emergency is actually occurring, wherein the response from the user comprises the user engaging with the wearable computing device or moving in a manner that moves the wearable computing device, and

wherein implementing the emergency response if the input is not received further comprises placing an emergency call an emergency telephone number and/or one or more contacts programmed in the wearable computing device and/or or one or more persons in a close proximity to the user.

11. The wearable computing device of any one of the preceding claims, wherein, before monitoring the oxygen saturation level of the user for the drop from above the upper threshold to consistently below the lower threshold, the plurality of operations further comprises ensuring the user is not asleep.

12. The wearable computing device of any one of the preceding claims, wherein the plurality of operations further comprises applying one or more signal quality check algorithms after determining the oxygen saturation drop above the certain magnitude and before requesting the input from the user via the wearable computing device to reduce false positives.

13. A method of detecting and responding to an oxygen emergency of a user of a wearable computing device, the method comprising:

detecting low motion of the user via the wearable computing device;

in response to detecting the low motion of the user, monitoring, via one or more photoplethysmography, PPG, sensors of the wearable computing device, an oxygen saturation level of the user for a drop from above an upper threshold to consistently below a lower threshold;

upon detecting the oxygen saturation level dropping from above the upper threshold to consistently below the lower threshold, determining, via the wearable computing device, an oxygen saturation drop above a certain magnitude;

in response to the oxygen saturation drop being above the certain magnitude, requesting an input from the user via the wearable computing device; and

implementing, via the wearable computing device, an emergency response if the input is not received.

14. The method of claim 13, wherein monitoring the oxygen saturation level of the user for the drop from above the upper threshold to consistently below the lower threshold further comprises:

employing a machine-learned model using the one or more PPG signals from the one or more PPG sensors that forecasts whether the oxygen saturation level of the user is consistently below the lower threshold over a specified duration while concurrently detecting intervals of the oxygen saturation level being above the upper threshold.

15. The method of claim 13 or 14 using the wearable device of any one of claims 1 to 12

FIG. 1A

FIG. 1B

150

100

| | | | |
|---|---|---|---|
| POWER COMPONENTS | MEMORY DEVICE 154 | EMITTER | 126 |
| 156 | | | 122 |
| INPUT/OUTPUT ELEMENT | CONTROLLER 152 | DETECTOR | 124 |
| 158 | | | |
| | DISPLAY 116 | DRIVER | 162 |
| WIRELESS COMPONENTS | | | |
| 160 | | | |
| | MOTION SENSOR(S) | BIOMETRIC SENSOR ELECTRODE(S) | 120 |
| | 164 | | |

NETWORK 166 ⟷ HOST COMPUTER 168

FIG. 2

TOUCH BUTTON AND/OR MOVE TO CANCEL

| 202 | 204 | 206 | 208 | 210 |
|---|---|---|---|---|
| **LOW MOTION DETECTION** | **LOW O2SAT DETECTION 1 - 2 MIN** | **O2SAT DROP DETECTION (RETROSPECTIVE)** | **GENTLE NUDGE AND COUNTDOWN 30 - 60s** | **CALLING & SHARING** |
| DETECT NON-ACTIVE AND TURN ON RED/IR PPG WHEN NEEDED | SpO2 DROPPING DROM HIGH AND CONSISTENTLY LOWER THAN 83% | >12% SpO2 DROPS COMPARED WITH NORMAL | SUSPECTED BREATHING EMERGENCY | ESCALATE AND CONNECT TO HELP |
| ACCELEROMETER SIGNALS AND/OR BAROMETRIC PRESSURE INDICATIVE OF HIGH PPG SIGNAL QUALITY | GREEN, RED AND IR PPG | GREEN, RED AND IR PPG (MULTIPATH PREFERRED) | USER INTERFACE (UX) | PHONE CALL |
| | TARGET: <4 FALSE POSITIVES / YR | TARGET: <2 FALSE POSITIVES / YR | TARGET: <1 FALSE POSITIVES / YR | TARGET: >70% MEAN SENSITIVITY |

200

212

*FIG. 3*

FIG. 4

FIG. 5

FIG. 6

AC COMPONENT VARIES OVER SHORT DURATIONS:
- REFLECTIONS FROM PULSATILE BLOOD, INCLUDING OXYGENATED AND DEOXYGENATED BLOOD CELLS
- NOISE INCLUDING BREATHING ARTIFACTS, MOTION ARTIFACTS AND AMBIENT LIGHT

DC COMPONENT IS CONSTANT OVER SHORT DURATIONS:
- REFLECTIONS FROM SKIN, BONE
- REFLECTIONS FROM NONPULSATILE BLOOD
- AMBIENT LIGHT
- NOISE

MEASURED PPG SIGNAL

TIME

EP 4 728 979 A1

400

O2SAT (%)

93 – – – – – – – – – –

402

83 – – – – – – – – – –

TIME

LATEST PPG SAMPLES
WITH BEST O2SAT

LAST FEW PPG SAMPLES
WITH LOW O2SAT

DNN

SpO2 DIFFERENCE > 12%?

*FIG. 7*

FIG. 8

EP 4 728 979 A1

FIG. 9

EP 4 728 979 A1

FIG. 10

300

DETECT LOW MOTION OF THE USER VIA THE WEARABLE COMPUTING DEVICE — 302

↓

IN RESPONSE TO DETECTING THE LOW MOTION OF THE USER, MONITOR, VIA THE WEARABLE COMPUTING DEVICE, AN OXYGEN SATURATION LEVEL OF THE USER FOR A DROP FROM ABOVE AN UPPER THRESHOLD TO CONSISTENTLY BELOW A LOWER THRESHOLD — 304

↓

UPON DETECT THE OXYGEN SATURATION LEVEL DROPPING FROM ABOVE THE UPPER THRESHOLD TO CONSISTENTLY BELOW THE LOWER THRESHOLD, DETERMINING, VIA THE WEARABLE COMPUTING DEVICE, AN OXYGEN SATURATION DROP ABOVE A CERTAIN MAGNITUDE — 306

↓

IN RESPONSE TO THE OXYGEN SATURATION DROP BEING ABOVE THE CERTAIN MAGNITUDE, REQUEST AN INPUT FROM THE USER VIA THE WEARABLE COMPUTING DEVICE — 308

↓

IMPLEMENT, VIA THE WEARABLE COMPUTING DEVICE, AN EMERGENCY RESPONSE IF THE INPUT IS NOT RECEIVED — 310

FIG. 11

# EUROPEAN SEARCH REPORT

**Application Number**

EP 25 20 8548

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/074701 A1 (HITE MICHAEL [US] ET AL) 7 March 2024 (2024-03-07) * abstract; figures 1-12 * * paragraphs [0204] - [0548] * ----- | 1-15 | INV. A61B5/024 A61B5/11 A61B5/1455 A61B5/00 G16H40/63 G16H50/20 |
| A | US 2024/298919 A1 (VALLAT RAPHAEL [FR] ET AL) 12 September 2024 (2024-09-12) * abstract; figures 1-10 * * paragraphs [0014] - [0234] * ----- | 1-15 | |
| A | LINGAMOORTHY ANUSH ET AL: "Dove: Shoulder-Based Opioid Overdose Detection and Reversal Device", 2023 IEEE/ACM CONFERENCE ON CONNECTED HEALTH: APPLICATIONS, SYSTEMS AND ENGINEERING TECHNOLOGIES (CHASE), ACM, 21 June 2023 (2023-06-21), pages 56-67, XP034382194, DOI: 10.1145/3580252.3586973 [retrieved on 2023-07-21] * Abstract; Introduction; Related work; Device design; System architecture;Evaluation; Discussion and future work; Conclusion * ----- | 1-15 | |

**TECHNICAL FIELDS SEARCHED (IPC)**

A61B
G16H

The present search report has been drawn up for all claims

| Place of search | Date of completion of the search | Examiner |
|---|---|---|
| The Hague | 11 February 2026 | Carta, Riccardo |

# EP 4 728 979 A1

## ANNEX TO THE EUROPEAN SEARCH REPORT
## ON EUROPEAN PATENT APPLICATION NO.

EP 25 20 8548

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2026

| Patent document cited in search report | Publication date | Patent family member(s) | Publication date |
|---|---|---|---|
| US 2024074701 A1 | 07-03-2024 | AU 2021385013 A1 | 25-05-2023 |
| | | CA 3195546 A1 | 27-05-2022 |
| | | EP 4236779 A1 | 06-09-2023 |
| | | US 2024074701 A1 | 07-03-2024 |
| | | WO 2022108712 A1 | 27-05-2022 |
| US 2024298919 A1 | 12-09-2024 | NONE | |

EPO FORM P0459

For more details about this annex : see Official Journal of the European Patent Office, No. 12/82

27